# EUROPEAN PATENT SPECIFICATION

(11) **EP 4 021 316 B1**
(45) Date of publication and mention of the grant of the patent: **14.02.2024**
(21) Application number: 20765457.5
(22) Date of filing: 25.08.2020
(51) Int. Cl.: A61B 17/29, A61B 17/32, A61B 18/14, A61B 90/00

(54) **FORCE LIMITING MECHANISM FOR SURGICAL INSTRUMENTS**
KRAFTBEGRENZUNGSMECHANISMUS FÜR CHIRURGISCHES INSTRUMENT
MÉCANISME DE LIMITATION DE FORCE POUR INSTRUMENTS CHIRURGICAUX

(30) Priority: 27.08.2019 US 201962892006 P; 13.09.2019 US 201962900104 P
(43) Date of publication of application: 06.07.2022
(73) Proprietor: CONMED Corporation, Largo, FL 33773 (US)
(72) Inventor: SZABO, Aaron, Swanton, OH 43558 (US)
(74) Representative: Strehl Schübel-Hopf & Partner
(86) International application number: PCT/US2020/047773
(87) International publication number: WO 2021/041398

(56) References cited:
- WO-A1-2019/157000
- US-A- 5 954 736
- US-A1- 2010 063 528
- US-A1- 2012 022 583
- US-A1- 2019 046 196

## Description

### CROSS-REFERENCE TO RELATED APPLICATIONS

The present application claims priority to U.S. Provisional No. 62/892006, filed on August 27, 2019 and U.S. Provisional No. 62/900104, filed on September 13, 2019.

### BACKGROUND OF THE INVENTION

### 1. FIELD OF THE INVENTION

The present invention relates to surgical instruments and, more specifically, to a force limiting mechanism for limiting the amount of force applied to the jaws of an electrosurgical instrument.

### 2. DESCRIPTION OF THE RELATED ART

Electrosurgical vessel sealers are used for the occlusion of blood vessels and halting of bleeding during surgical procedures. The electrodes of the vessel sealer are carried by a pair of opposing jaws and interconnected to an electrosurgical generator that can selectively supply radiofrequency (RF) energy to the electrodes. A user may close the jaws around a vessel to be sealed by squeezing a lever associated with a handle assembly. The vessel may then be sealed by supplying the RF energy to the clamped vessel. A moveable blade may be additionally incorporated into the jaws for cutting of the sealed blood vessel along an intermediate portion of the seal created by the energized electrodes in response to user activation of a second trigger.

One problem that arises in the use of electrosurgical vessel sealers is the user applying too much force to the jaw closing lever, which can result in breakage of the device. Accordingly, there is a need in the art for an approach that can limit the amount of force that a user can apply to the jaws via the handle lever.

US 2019/046196 A1 discloses a surgical device with a conventional overload mechanism. Other conventional solutions can be found in US 5 954 736 A, WO 2019/157000 A1, US 2010/063528 A1, and US 2012/022583 A1.

### BRIEF SUMMARY OF THE INVENTION

The present invention limits the amount of force that a user can apply to the jaws of a surgical instrument by decoupling the handle lever from the drive shaft if the handle lever is moved beyond the closed position. More specifically, the present invention is a surgical instrument as defined in claim 1, said surgical instrument comprising a body having a drive shaft extending along a longitudinal axis and coupled to a pair of jaws that are moveable between an open and a closed position, a lever bearing secured around the drive shaft for movement therewith and having a stop extending therefrom, a first lever having a first upper end pivotally mounted within the body and a first lower end that extends out of the body, a second lever having a second upper end interconnected to the first upper end to the first lever and extending to a second lower end, wherein the second lever engages the stop of the lever bearing, and a spring extending between an intermediation portion of the first lever and the second lower end of the second lever. The second lever may include a pair of bearing surfaces in contact with the stop of the lever bearing.

In one embodiment, the surgical instrument may further comprise a link pivotally interconnected to the first upper end of the first lever and to the second upper end of the second lever. The second lever may include a pair of bearing surfaces in contact with the stop of the lever bearing. In this embodiment, the spring may be configured to provide a force biasing the first lever and the second lever together. The first lever may be moveable between a first position where the first lever causes the second lever to position that lever bearing so that the drive shaft puts the jaws in the open position, a second position where the first lever causes the second lever to position the lever bearing position so that the drive shaft puts the jaws in the closed position, and a third position where the first lever has separated from the second lever against the bias of the spring so that the second lever and the lever bearing do not move the drive shaft and the jaws remain in the closed position. A pair of tabs may extend between a first set of posts positioned on the first lever and a second set of posts positioned in the second lever that set a predetermined minimum distance between the first lever and the second lever.

In another embodiment, the first lever and the second lever may be pivotally mounted within the body about a common pivot point. In this embodiment, the spring is configured to provide a force biasing the first lever and the second lever apart. The first lever is moveable between a first position where the first lever causes the second lever to position that lever bearing so that the drive shaft puts the jaws in the open position, a second position where the first lever causes the second lever to position the lever bearing position so that the drive shaft puts the jaws in the closed position, and a third position where the first lever is moved closer to the second lever against the bias of the spring so that the second lever and the lever bearing do not move the drive shaft and the jaws remain in the closed position.

In a further embodiment, the present invention comprises a non-surgical method of limiting the amount of force applied by a user to the jaws of a surgical instrument as defined in claim 10. The method includes the steps of providing a first lever extending from a body of the surgical instrument for movement by a user to close the jaws of the surgical instrument, providing a second lever that is responsive to movement of the first lever to translate a lever bearing axially along a longitudinal axis of the body of the surgical instrument, and interconnecting the first lever and the second lever with a spring that allows for movement of the second lever along with the first lever when the first lever is moved to a position that results in closing of the jaws of the surgical instrument but prevents movement of the second lever when the first lever is moved beyond the closing of the jaws so that movement of the first lever past the position that results in closing of the jaws. The first lever and the second lever are interconnected by a link that is pivotally connected to both the first lever and the second lever, with the spring being configured to bias the first lever and the second lever toward each other. The first lever and the second lever may instead be pivotally mounted in the body to a common pivot point, with the spring configured to bias the first lever and the second lever away from each other.

### BRIEF DESCRIPTION OF THE SEVERAL VIEWS OF THE DRAWING(S)

The present invention will be more fully understood and appreciated by reading the following Detailed Description in conjunction with the accompanying drawings, in which:
FIG. 1 is a schematic of an electrosurgical system having a pair of jaws carrying electrodes for electrosurgically treating tissue
FIG. 2 is an isometric view of the components of a handle assembly for an electrosurgical vessel sealer according to the present invention;
FIG. 3 is a front view of the components of a partially disassembled handle assembly for an electrosurgical vessel sealer according to the present invention;
FIG. 4A is a first isometric view of a handle and force limiting mechanism according to the present invention;
FIG. 4B is a second isometric view of a handle and force limiting mechanism according to the present invention;
FIG. 5 is an isometric view of a force limiting mechanism according to the present invention;
FIG. 6 is a front view of the handle assembly when the jaws of the electrosurgical vessel sealer are in the open position;
FIG. 7 is a front view of handle assembly when the jaws of the electrosurgical vessel sealer are in the closed position; and
FIG. 8 is a front view of the handle assembly when the jaws of the electrosurgical vessel sealer are in the closed position and the handle lever has been moved beyond the point necessary to close the laws.
FIG. 9 is an isometric view of the components of a second embodiment of a handle assembly for an electrosurgical vessel sealer according to the present invention;
FIG. 10 is an isometric view of a force limiting mechanism according to a second embodiment of the present invention;
FIG. 11A is a side view of a handle and force limiting mechanism in a first position according to a second embodiment of the present invention;
FIG. 11B is a side view of a handle and force limiting mechanism according to a second embodiment of the present invention;
FIG. 12A is a side view of a handle and force limiting mechanism according to a second embodiment of the present invention;
FIG. 12B is an isometric view of a handle and force limiting mechanism in a second position according to a second embodiment of the present invention;
FIG. 13 is an isometric view of a handle lever and force limiting assembly according to a second embodiment of the present invention;
FIG. 14 is a front view of the handle assembly when the jaws of a second embodiment of the electrosurgical vessel sealer are in the open position;
FIG. 15 is a front view of handle assembly when the jaws of a second embodiment of the electrosurgical vessel sealer are in the closed position; and
FIG. 16 is a front view of the handle assembly when the jaws of a second embodiment of the electrosurgical vessel sealer are in closed position and the handle lever has been moved beyond the point necessary to close the laws.

### DETAILED DESCRIPTION OF THE INVENTION

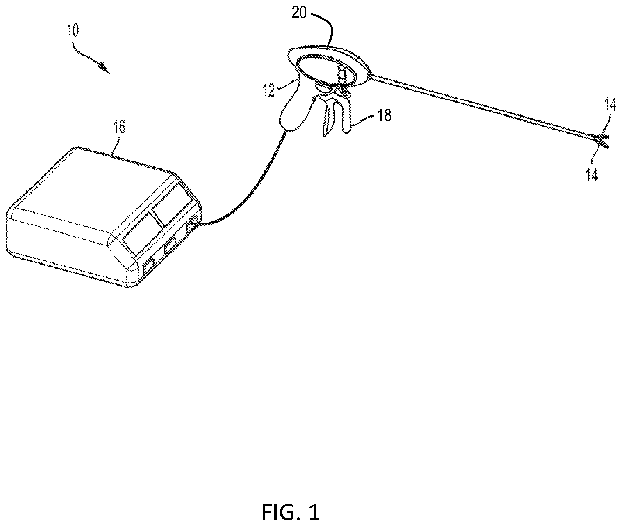

Referring to the figures, wherein like numeral refer to like parts throughout, there is seen in FIG. 1 a vessel sealing system 10 comprising a vessel sealer 12 having a pair of conductive opposing jaws 14 that are interconnected to an electrosurgical generator 16 that can supply RF energy to electrodes of jaws 14 for the desiccation of a blood vessel trapped between jaw 14. The dimensions of jaws 14 and the type of RF energy supplied will produce desiccation of the blood vessel in a region of a particular width as determined by the thermal spread of the energy being supplied to the blood vessel. As is known in the art, jaws 14 are pivotally mounted to vessel sealer 12 for movement between an open position and a closed position in response to a user operating a lever 18 that extends from the main body 20 of sealer 12 and can be gripped by a user.

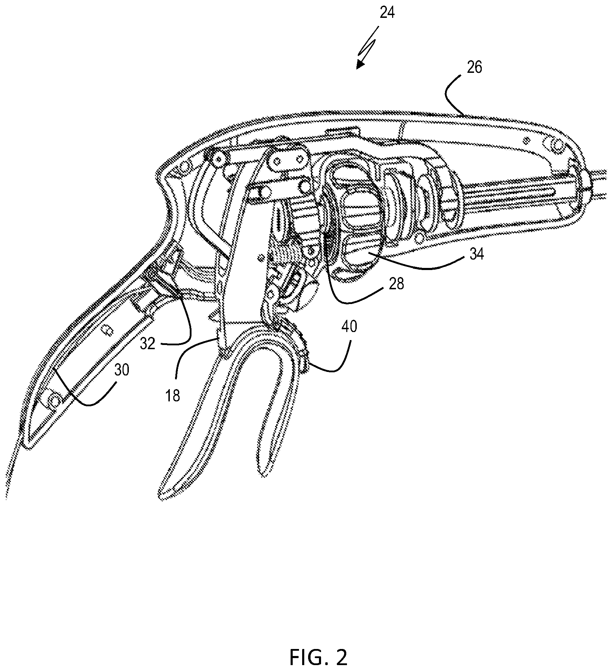

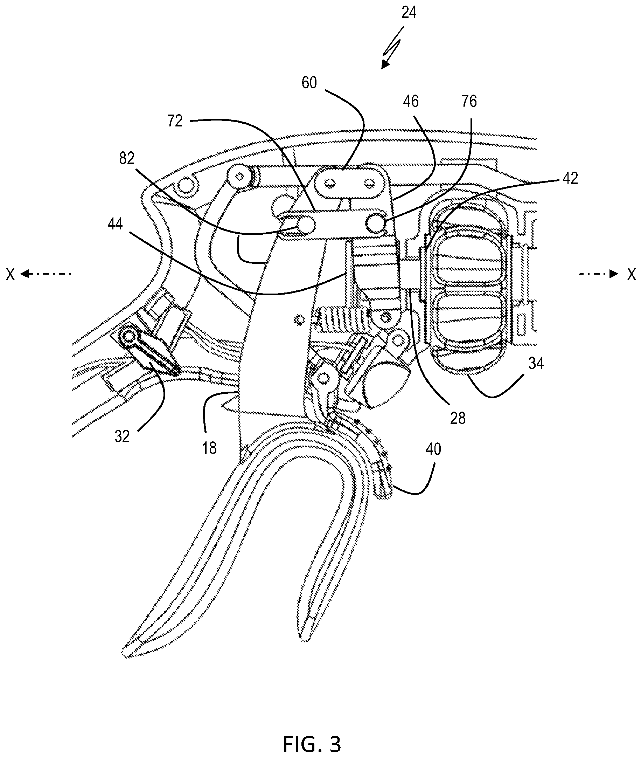

Referring to FIGS. 2 and 3, vessel sealer 12 includes a handle assembly 24 comprised of a housing body 26 that encloses a drive shaft 28 that extends from body 26 and drives a longitudinal axis X-X of vessel sealer 12. Drive shaft 28 is coupled to jaws 14 so that longitudinal movement of drive shaft 28 will mechanically move jaws 14 between the open and closed positioned. Housing body 26 also encloses cabling 30 for delivering energy to jaws 14 as well as a latching mechanism 32 for selectively retaining lever 18 when it is moved from a first position, where jaws 14 are open, to a second position, where jaws 14 are closed. Housing body 26 partially encloses a knob 34 that is coupled to an outer shaft that surrounds drive shaft 28 and supports jaws 14 such that rotation of knob 34 will rotate jaws 14 through 360 degrees. Handle assembly 24 further comprises a knife trigger 40 for extending a blade (not shown) between jaws 14 to sever a treated vessel.

A lever bearing having a distal stop 42 and a proximal stop 44 is secured to drive shaft 28 so that movement of lever bearing causes longitudinal translation of drive shaft 28 and thus opening and closing of jaws 14. Lever bearing is rotatable through 360 degrees along with drive shaft 28 when a user rotates knob 34 so that jaws 14 can be positioned as desired. Lever 18 is interconnected to lever bearing for movement of drive shaft 28 by a secondary lever 46, illustrated as having a H-shaped body with lower legs 48 extending on either side of drive shaft 28 and having proximal bearing surfaces 50 and 52 in engagement with proximal stop 44. Bearing surfaces 50 and 52 will remain in engagement with proximal stop 44 regarding of any rotation of drive shaft 28. As a result, jaws 14 may be closed via operation of lever 18 regardless of any rotation of jaws 14 by knob 34, thereby allowing a user to be able to close jaws 14 in any position.

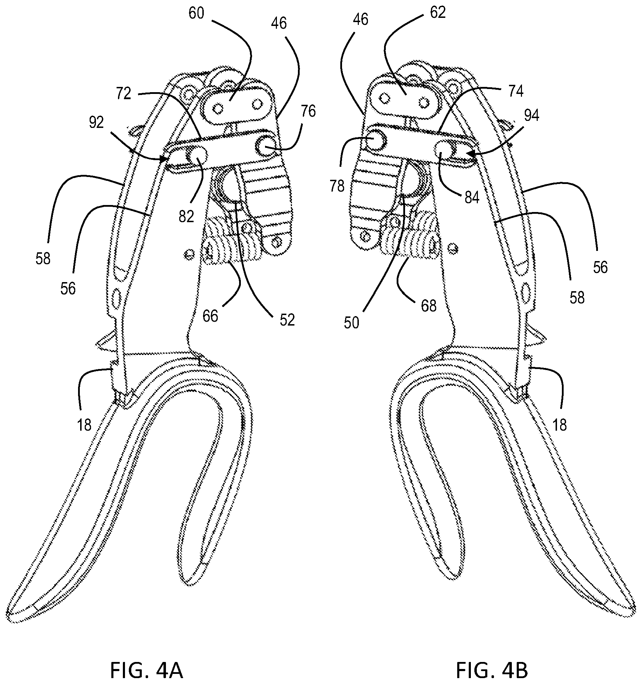

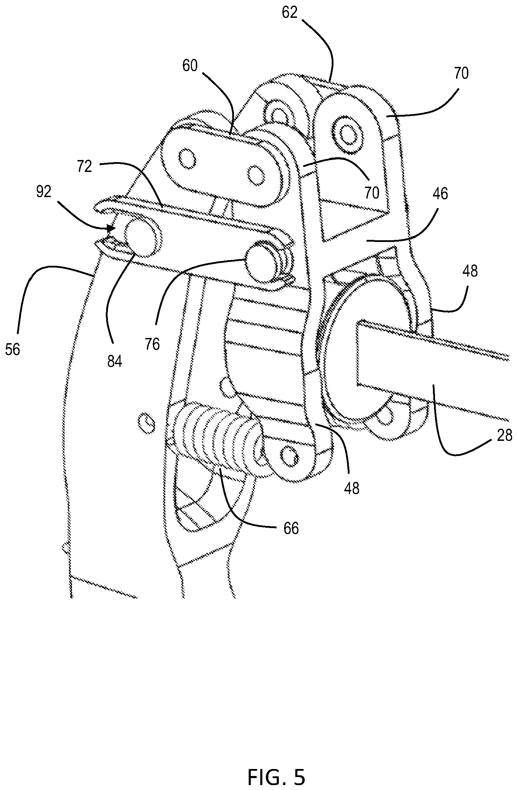

Lever 18 has an upper end positioned within body 26 and forming a pair of opposing tines 56 and 58 that extend on either side of longitudinal axis X-X. Each of opposing tines 56 and 58 is pivotally coupled to the upper ends of secondary lever 46 by one of a corresponding pair of links 60 and 62. A pair of springs 66 and 68 are coupled between the lower ends of secondary lever 46 and the base of tines 56 and 58, respectively. Springs 66 and 68 are configured to have a preload providing a predetermined biasing force that holds lever 18 and secondary lever 46 together so that pivoting of lever 18 by a user will pull secondary lever against proximal stop 44. A pair of positioning tabs 72 and 74 are pivotally connected to a second pair of posts 76 and 78 positioned on secondary lever 46 and to a first pair of posts 82 and 84 positioned on tines 56 and 58. As seen in FIGS. 4A, 4B, and 5, each of tabs 72 and 74 may be coupled to second set of posts 84 and 86 using slots 92 and 94 so that lever 18 can pivot relative to secondary lever 46 if the preload of springs 66 and 68 is overcome. Positioning tabs 72 and 74 maintain a minimum distance between lever 18 and secondary lever 46 against the preload force of springs 66 and 68 when a user is not applying a force to lever 18.

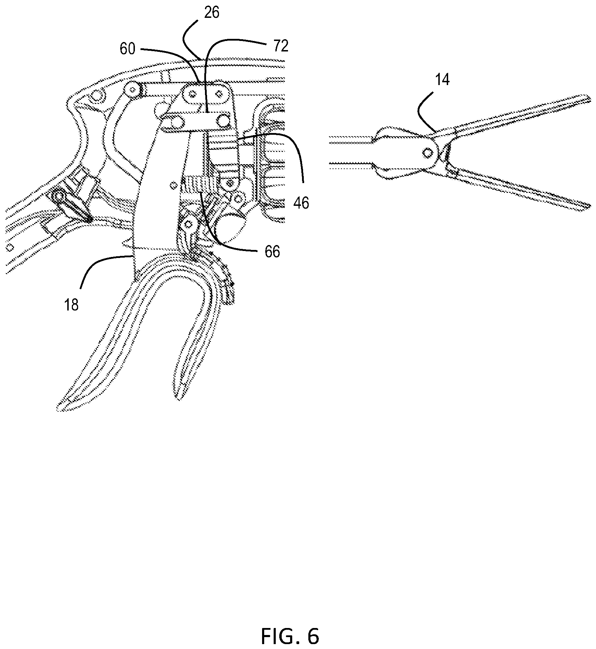

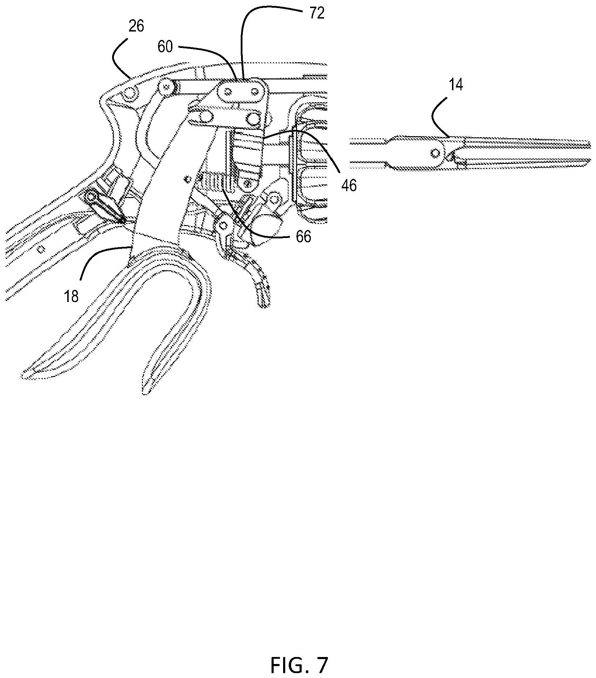

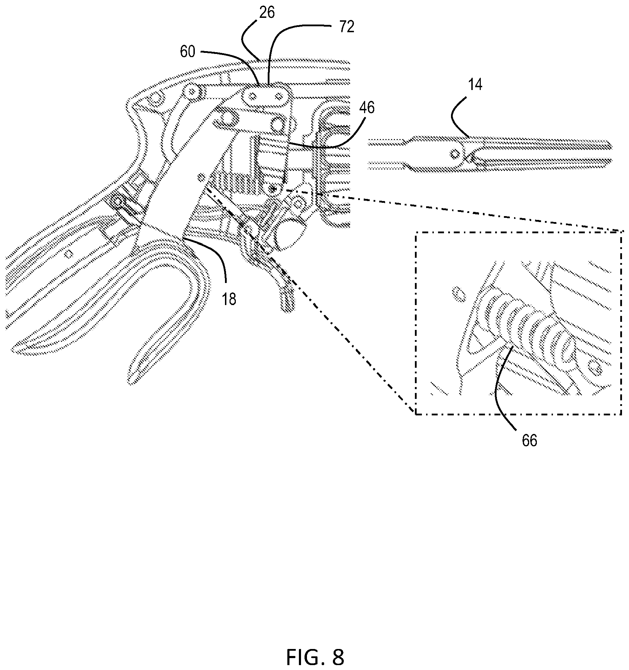

Referring to FIG. 6, when lever 18 is in a first position, jaws 14 will be open. As lever 18 is pivoted into a second position, as seen in FIG. 7, secondary lever 46 will pivot with lever 18 under the preload of springs 66 and 68. Pivoting of secondary lever 46 causes bearing surfaces 50 and 52 to push against proximal stop 44 thereby moving lever bearing axially along longitudinal axis X-X so that jaws 14 are moved into the closed position. Referring to FIG. 8, any further application of force to lever 18 will overcome the preload of springs 66 and 68 so that spring 66 and 68 lengthen, thereby allowing lever 18 to pivot independently of secondary lever 46, which remains stationary. As a result, the further application of force is not transmitted to drive shaft 28.

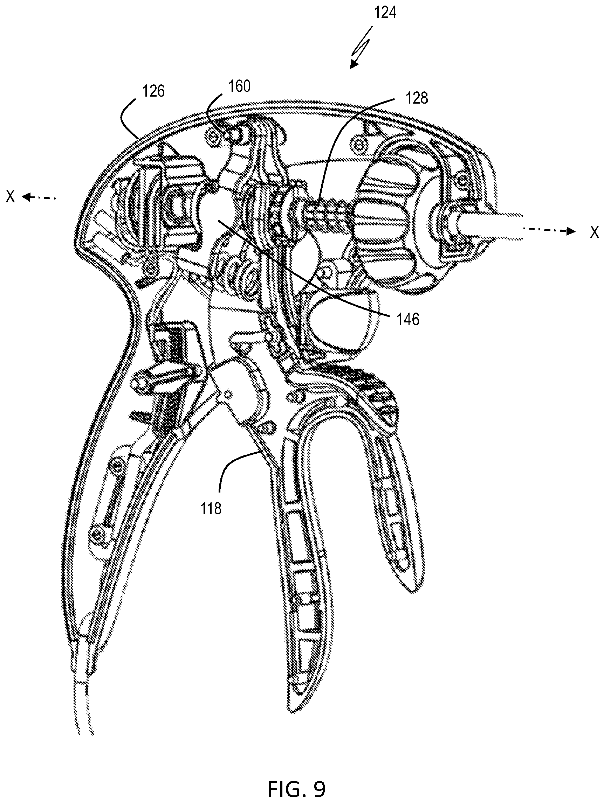

There is seen in FIG. 9, another embodiment of a handle assembly 124 comprised of a housing body 126 that encloses a drive shaft 128 that extends from body 126 and extends along longitudinal axis X-X of vessel sealer 12. In this embodiment, lever 118 is pivotally mounted at an upper end to a fixed location within housing body 126 and extends out of body 126 for grasping by a user. Lever 118 is coupled to drive shaft 128 via a secondary lever 146 that is mounted to a common pivot point and interconnected to lever 118 by a spring 166.

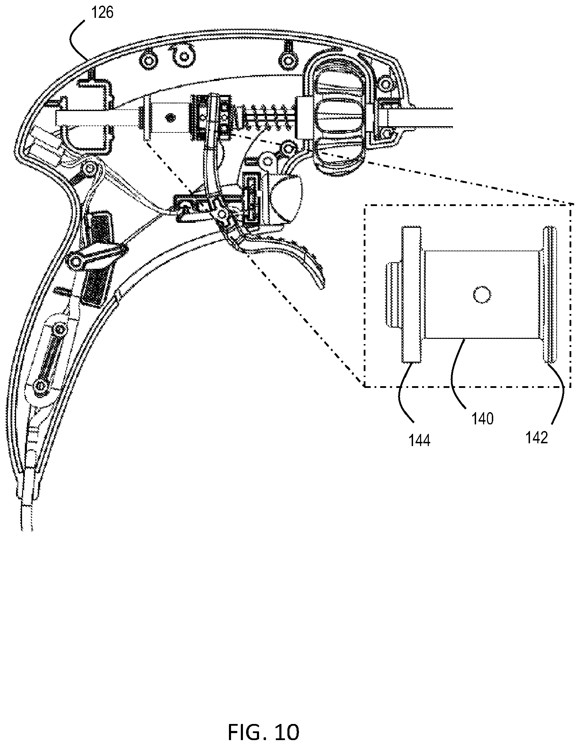

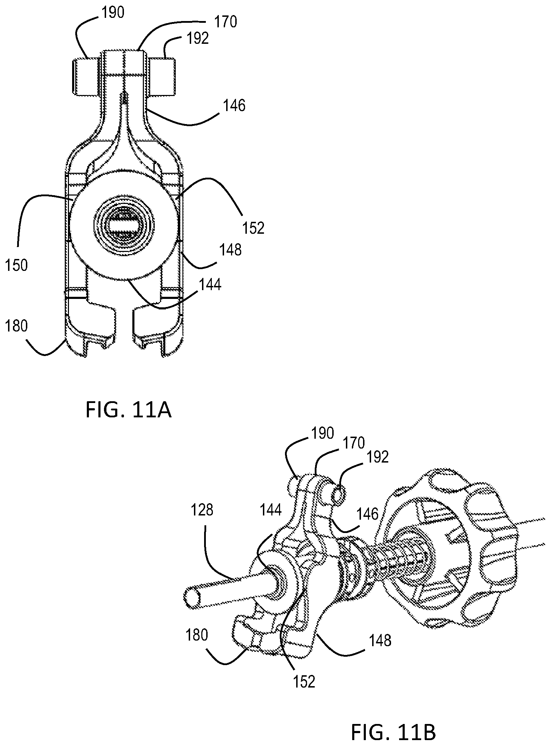

Referring to FIG. 10, a lever bearing 140 having a distal stop 142 and a proximal stop 144 is secured to drive shaft 128 so that movement of lever bearing 140 causes longitudinal translation of drive shaft 128 and thus opening and closing of jaws 14. As seen in FIGS. 11A and 11B, secondary lever 146 has an upper end 170 that can accept a pivot pin 160 for pivotally mounting secondary lever 146 within housing body 126. Upper end 170 may further include bearings 190 and 192 for pivotally coupling to lever 118 so that that lever 118 and secondary lever 146 are mounted to a common pivot point. Secondary lever 146 includes a pair of lower legs 148 that extend around lever bearing 140. Legs 148 include proximal bearing surfaces 150 and 152 for engaging proximal stop 144. Pivotal movement of secondary lever 146 will cause proximal bearing surfaces 150 and 152 to move proximal stop 144 and lever bearing 140 axially, thereby moving drive shaft axially along axis X-X. Lower legs 148 cooperate to define a spring holder 180 for accepting spring 166.

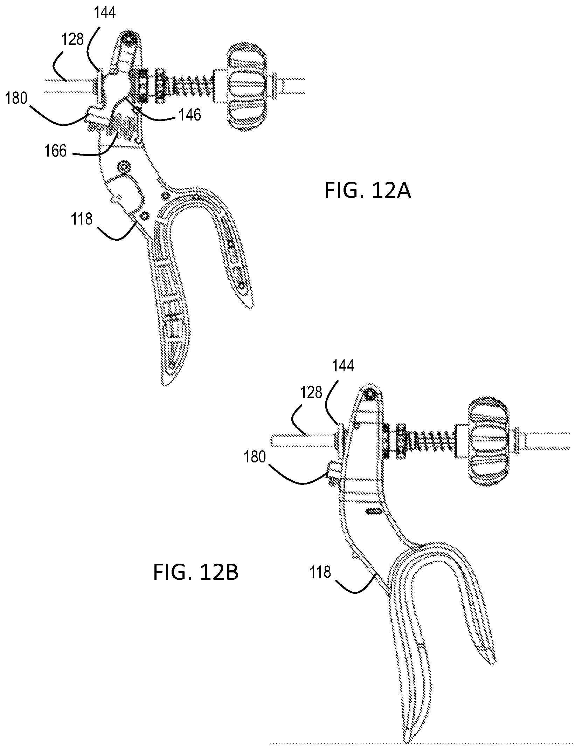

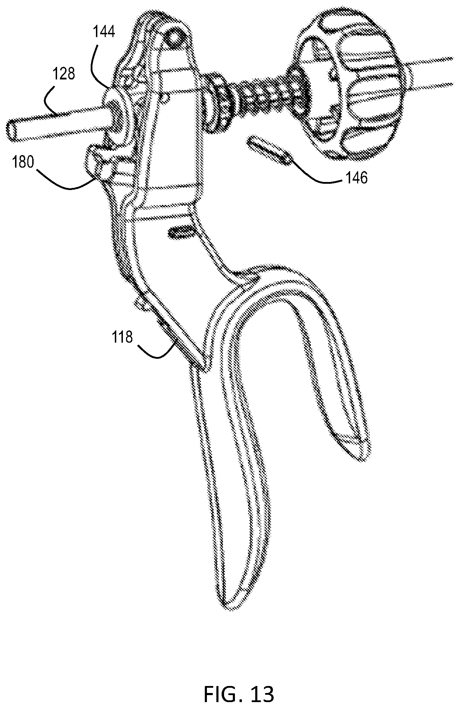

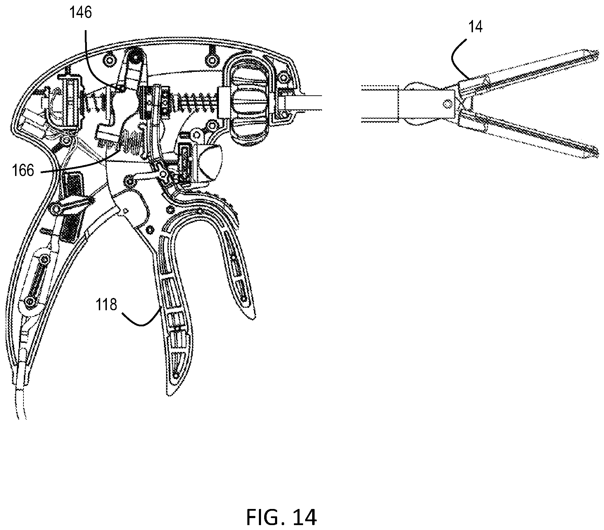

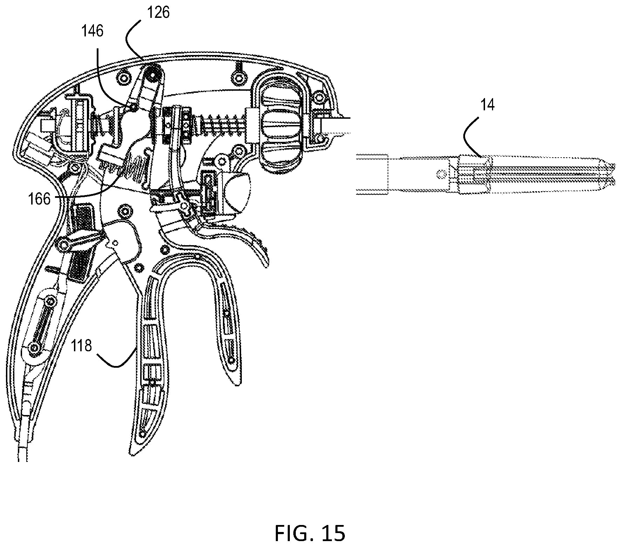

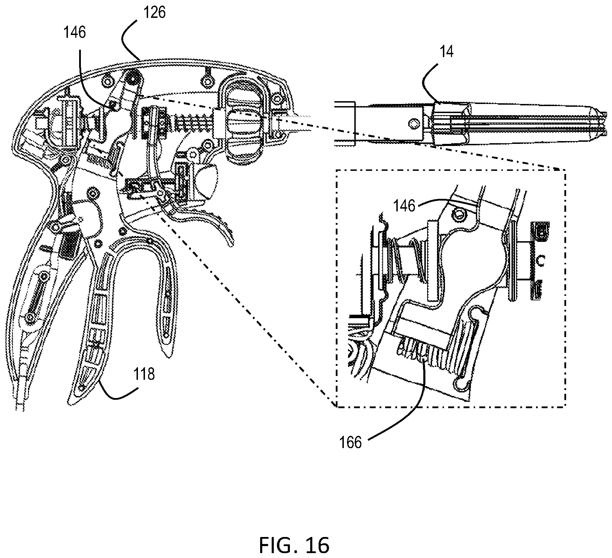

Referring to FIGS. 12A and 12B, lever 118 comprises two halves 118a and 118b that are closed about spring 166 and secondary lever 146 when handle body 126 is assembled. As seen in FIG. 13, a preload pin 182 may be used to partially compress spring 166 when installed so that spring will not compress when lever 118 is moved from the first position, where jaws 14 are open as seen in FIG. 14, to the second position, where jaws 14 are closed as seen in FIG. 15. Instead, spring 166 will only further compress when jaws 14 are already closed and a user continues to apply a force to lever 118, as seen in FIG. 16. As a result, any force applied to lever 118 after jaws have closed will be absorbed by spring 166 and not be communicated to jaws 14.

## Claims

1. A surgical instrument, comprising:
a body (26; 126) having a drive shaft (28; 128) extending along a longitudinal axis (X) and coupled to a pair of jaws (14) that are moveable between an open and a closed position;
a lever bearing (140) secured around the drive shaft (28; 128) for movement therewith and having a stop (42, 44; 142, 144) extending therefrom;
a first lever (18; 118) having a first upper end pivotally mounted within the body (26; 126) and a first lower end that extends out of the body (26; 126);
a second lever (46; 146) having a second upper end interconnected to the first upper end of the first lever (18; 118) and extending to a second lower end, wherein the second lever (46; 146) engages the stop (42, 44; 142, 144) of the lever bearing (140); and
a spring (66; 166) extending between an intermediation portion of the first lever (18; 118) and the second lower end of the second lever (46; 146).

2. The surgical instrument of claim 1, wherein the second lever (46; 146) includes pair of bearing surfaces (50, 52; 150, 152) in contact with the stop (42, 44; 142, 144) of the lever bearing (140)

3. The surgical instrument of claim 2, further comprising a link (60, 62) pivotally interconnected to the first upper end of the first lever (18) and to the second upper end of the second lever (46).

4. The surgical instrument of claim 3, wherein the spring (66) is configured to provide a force biasing the first lever (18) and the second lever (46) together.

5. The surgical instrument of claim 4, wherein the first lever (18) is moveable between
a first position where the first lever (18) causes the second lever (46) to position the lever bearing so that the drive shaft (28) puts the jaws (14) in the open position,
a second position where the first lever (18) causes the second lever (46) to position the lever bearing position so that the drive shaft (28) puts the jaws (14) in the closed position, and
a third position where the first lever (18) has separated from the second lever (46) against the bias of the spring (66) so that the second lever (46) and the lever bearing do not move the drive shaft (28) and the jaws (14) remain in the closed position.

6. The surgical instrument of claim 5, further comprising a pair of tabs (72, 74) extending between a first set of posts (78, 80) positioned on the first lever (18) and a second set of posts (76, 78) positioned in the second lever (46) that set a predetermined minimum distance between the first lever (18) and the second lever (46).

7. The surgical instrument of claim 2, wherein the first lever (118) and the second lever (146) are pivotally mounted within the body (126) about a common pivot point.

8. The surgical instrument of claim 7, wherein the spring (166) is configured to provide a force biasing the first lever (118) and the second lever (146) apart.

9. The surgical instrument of claim 8, wherein the first lever (118) is moveable between
a first position where the first lever (118) causes the second lever (146) to position the lever bearing (140) so that the drive shaft (128) puts the jaws (14) in the open position,
a second position where the first lever (118) causes the second lever (146) to position the lever bearing position so that the drive shaft (128) puts the jaws (14) in the closed position, and
a third position where the first lever (118) is moved closer to the second lever (146) against the bias of the spring (166) so that the second lever (146) and the lever bearing (140) do not move the drive shaft (128) and the jaws (14) remain in the closed position.

10. A non-surgical method of limiting the amount of force applied by a user to the jaws (14) of a surgical instrument, comprising the steps of:
providing a first lever (18) extending from a body (26) of the surgical instrument for movement by a user to close the jaws (14) of the surgical instrument;
providing a second lever (46) that is responsive to movement of the first lever (18) to translate a lever bearing (140) axially along a longitudinal axis (X) of the body (26) of the surgical instrument;
interconnecting the first lever (18) and the second lever (46) with a spring (66) that allows for movement of the second lever (46) along with the first lever (18) when the first lever (18) is moved to a position that results in closing of the jaws (14) of the surgical instrument but prevents movement of the second lever (46) when the first lever (18) is moved beyond the closing of the jaws (14) so that movement of the first lever (18) past the position results in closing of the jaws (14),
wherein the first lever (18) and the second lever (46) are interconnected by a link (60, 62) that is pivotally connected to both the first lever (18) and the second lever (46), and
wherein the spring (66) is configured to bias the first lever (18) and the second lever (46) toward each other.

## Patentansprüche

1. Chirurgisches Instrument, umfassend:
einen Körper (26; 126) mit einer Antriebswelle (28; 128), die sich entlang einer Längsachse (X) erstreckt und mit einem Paar von Backen (14) gekoppelt ist, die zwischen einer offenen und einer geschlossenen Stellung beweglich sind;
ein Hebellager (140), das um die Antriebswelle (28; 128) herum befestigt ist, so dass es sich mit ihr bewegen kann, und das einen Anschlag (42, 44; 142, 144) aufweist, der sich von ihm aus erstreckt;
einen ersten Hebel (18; 118) mit einem ersten oberen Ende, das schwenkbar in dem Körper (26; 126) angebracht ist, und einem ersten unteren Ende, das aus dem Körper (26; 126) herausragt;
einen zweiten Hebel (46; 146) mit einem zweiten oberen Ende, das mit dem ersten oberen Ende des ersten Hebels (18; 118) verbunden ist und sich zu einem zweiten unteren Ende erstreckt, wobei der zweite Hebel (46; 146) mit dem Anschlag (42, 44; 142, 144) des Hebellagers (140) in Eingriff steht; und
eine Feder (66; 166), die sich zwischen einem Mittelabschnitt des ersten Hebels (18; 118) und dem zweiten unteren Ende des zweiten Hebels (46; 146) erstreckt.

2. Chirurgisches Instrument nach Anspruch 1, wobei der zweite Hebel (46; 146) ein Paar Lagerflächen (50, 52; 150, 152) aufweist, die mit dem Anschlag (42, 44; 142, 144) des Hebellagers (140) in Kontakt stehen.

3. Chirurgisches Instrument nach Anspruch 2, das ferner ein Verbindungsglied (60, 62) umfasst, das schwenkbar mit dem ersten oberen Ende des ersten Hebels (18) und dem zweiten oberen Ende des zweiten Hebels (46) verbunden ist.

4. Chirurgisches Instrument nach Anspruch 3, wobei die Feder (66) so konfiguriert ist, dass sie eine Kraft bereitstellt, die den ersten Hebel (18) und den zweiten Hebel (46) zusammendrückt.

5. Chirurgisches Instrument nach Anspruch 4, wobei der erste Hebel (18) beweglich ist zwischen
einer ersten Stellung, in der der erste Hebel (18) den zweiten Hebel (46) veranlasst, das Hebellager so zu positionieren, dass die Antriebswelle (28) die Backen (14) in die offene Stellung bringt,
einer zweiten Stellung, in der der erste Hebel (18) den zweiten Hebel (46) veranlasst, die Hebellagerposition so zu positionieren, dass die Antriebswelle (28) die Backen (14) in die geschlossene Stellung bringt, und
einer dritten Stellung, in der sich der erste Hebel (18) von dem zweiten Hebel (46) gegen die Vorspannung der Feder (66) getrennt hat, so dass der zweite Hebel (46) und das Hebellager die Antriebswelle (28) nicht bewegen und die Backen (14) in der geschlossenen Stellung bleiben.

6. Chirurgisches Instrument nach Anspruch 5, das ferner ein Paar Laschen (72, 74) umfasst, die sich zwischen einem ersten Satz von Pfosten (78, 80), die auf dem ersten Hebel (18) positioniert sind, und einem zweiten Satz von Pfosten (76, 78), die in dem zweiten Hebel (46) positioniert sind, erstrecken und einen vorbestimmten Mindestabstand zwischen dem ersten Hebel (18) und dem zweiten Hebel (46) festlegen.

7. Chirurgisches Instrument nach Anspruch 2, wobei der erste Hebel (118) und der zweite Hebel (146) innerhalb des Körpers (126) um einen gemeinsamen Drehpunkt schwenkbar gelagert sind.

8. Chirurgisches Instrument nach Anspruch 7, wobei die Feder (166) dazu konfiguriert ist, eine Kraft bereitzustellen, die den ersten Hebel (118) und den zweiten Hebel (146) auseinanderdrückt.

9. Chirurgisches Instrument nach Anspruch 8, wobei der erste Hebel (118) beweglich ist zwischen
einer ersten Stellung, in der der erste Hebel (118) den zweiten Hebel (146) veranlasst, das Hebellager (140) so zu positionieren, dass die Antriebswelle (128) die Backen (14) in die offene Stellung bringt,
einer zweiten Stellung, in der der erste Hebel (118) den zweiten Hebel (146) veranlasst, die Hebellagerposition so zu positionieren, dass die Antriebswelle (128) die Backen (14) in die geschlossene Stellung bringt, und
einer dritten Stellung, in der der erste Hebel (118) gegen die Vorspannung der Feder (166) näher an den zweiten Hebel (146) bewegt wird, so dass der zweite Hebel (146) und das Hebellager (140) die Antriebswelle (128) nicht bewegen und die Backen (14) in der geschlossenen Stellung bleiben.

10. Nicht-chirurgisches Verfahren zur Begrenzung der von einem Benutzer auf die Backen (14) eines chirurgischen Instruments ausgeübten Kraft, das die folgenden Schritte umfasst:
Bereitstellen eines ersten Hebels (18), der sich von einem Körper (26) des chirurgischen Instruments erstreckt, um von einem Benutzer zum Schließen der Backen (14) des chirurgischen Instruments bewegt zu werden;
Bereitstellen eines zweiten Hebels (46), der auf die Bewegung des ersten Hebels (18) anspricht, um ein Hebellager (140) axial entlang einer Längsachse (X) des Körpers (26) des chirurgischen Instruments zu verschieben;
Verbinden des ersten Hebels (18) und des zweiten Hebels (46) mit einer Feder (66), die eine Bewegung des zweiten Hebels (46) zusammen mit dem ersten Hebel (18) ermöglicht, wenn der erste Hebel (18) in eine Stellung bewegt wird, die zu einem Schließen der Backen (14) des chirurgischen Instruments führt, aber eine Bewegung des zweiten Hebels (46) verhindert, wenn der erste Hebel (18) über das Schließen der Backen (14) hinaus bewegt wird, so dass eine Bewegung des ersten Hebels (18) über die Stellung hinaus zu einem Schließen der Backen (14) führt,
wobei der erste Hebel (18) und der zweite Hebel (46) durch ein Verbindungsglied (60, 62) miteinander verbunden sind, das sowohl mit dem ersten Hebel (18) als auch mit dem zweiten Hebel (46) schwenkbar verbunden ist, und
wobei die Feder (66) dazu konfiguriert ist, den ersten Hebel (18) und den zweiten Hebel (46) gegeneinander vorzuspannen.

## Revendications

1. Instrument chirurgical, comprenant :
un corps (26 ; 126) ayant un arbre (28 ; 128) d'entraînement s'étendant le long d'un axe longitudinal (X) et couplé à une paire de mâchoires (14) qui sont mobiles entre une position ouverte et une position fermée ;
un support (140) de levier fixé autour de l'arbre (28 ; 128) d'entraînement pour un mouvement avec celui-ci et ayant une butée (42, 44 ; 142, 144) s'étendant depuis celui-ci ;
un premier levier (18 ; 118) ayant une première extrémité supérieure montée pivotante à l'intérieur du corps (26 ; 126) et une première extrémité inférieure qui s'étend hors du corps (26 ; 126) ;
un deuxième levier (46 ; 146) ayant une deuxième extrémité supérieure interconnectée à la première extrémité supérieure du premier levier (18 ; 118) et s'étendant jusqu'à une deuxième extrémité inférieure, dans lequel le deuxième levier (46 ; 146) engage la butée (42, 44 ; 142, 144) du support (140) de levier ; et
un ressort (66 ; 166) s'étendant entre une partie intermédiaire du premier levier (18 ; 118) et la deuxième extrémité inférieure du deuxième levier (46 ; 146).

2. Instrument chirurgical selon la revendication 1, dans lequel le deuxième levier (46 ; 146) inclut une paire de surfaces d'appui (50, 52 ; 150, 152) en contact avec la butée (42, 44 ; 142, 144) du support (140) de levier.

3. Instrument chirurgical selon la revendication 2, comprenant en outre une liaison (60, 62) interconnectée pivotante à la première extrémité supérieure du premier levier (18) et à la deuxième extrémité supérieure du deuxième levier (46).

4. Instrument chirurgical selon la revendication 3, dans lequel le ressort (66) est configuré pour fournir une force poussant le premier levier (18) et le deuxième levier (46) ensemble.

5. Instrument chirurgical selon la revendication 4, dans lequel le premier levier (18) est mobile entre
une première position où le premier levier (18) amène le deuxième levier (46) à positionner le support de levier de telle façon que l'arbre (28) d'entraînement met les mâchoires (14) dans la position ouverte,
une deuxième position où le premier levier (18) amène le deuxième levier (46) à positionner la position de support de levier de telle façon que l'arbre (28) d'entraînement met les mâchoires (14) dans la position fermée, et
une troisième position où le premier levier (18) s'est séparé du deuxième levier (46) contre la poussée du ressort (66) de telle façon que le deuxième levier (46) et le support de levier ne déplace pas l'arbre (28) d'entraînement et les mâchoires (14) restent dans la position fermée.

6. Instrument chirurgical selon la revendication 5, comprenant en outre une paire de languettes (72, 74) s'étendant entre un premier ensemble de montants (78, 80) positionnés sur le premier levier (18) et un deuxième ensemble de montants (76, 78) positionnés dans le deuxième levier (46) qui définissent une distance minimum prédéterminée entre le premier levier (18) et le deuxième levier (46).

7. Instrument chirurgical selon la revendication 2, dans lequel le premier levier (118) et le deuxième levier (146) sont montés pivotants à l'intérieur du corps (126) autour d'un point de pivot commun.

8. Instrument chirurgical selon la revendication 7, dans lequel le ressort (166) est configuré pour fournir une force poussant le premier levier (118) et le deuxième levier (146) à l'écart l'un de l'autre.

9. Instrument chirurgical selon la revendication 8, dans lequel le premier levier (118) est mobile entre
une première position où le premier levier (118) amène le deuxième levier (146) à positionner le support (140) de levier de telle façon que l'arbre (128) d'entraînement met les mâchoires (14) dans la position ouverte,
une deuxième position où le premier levier (118) amène le deuxième levier (146) à positionner la position de support de levier de telle façon que l'arbre (128) d'entraînement met les mâchoires (14) dans la position fermée, et
une troisième position où le premier levier (118) est rapproché du deuxième levier (146) contre la poussée du ressort (166) de telle façon que le deuxième levier (146) et le support (140) de levier ne déplace pas l'arbre (128) d'entraînement et les mâchoires (14) restent dans la position fermée.

10. Procédé non chirurgical de limitation de la quantité de force appliquée par un utilisateur aux mâchoires (14) d'un instrument chirurgical, comprenant les étapes de :
prévision d'un premier levier (18) s'étendant depuis un corps (26) de l'instrument chirurgical pour un mouvement par un utilisateur pour fermer les mâchoires (14) de l'instrument chirurgical ;
prévision d'un deuxième levier (46) qui réagit à un mouvement du premier levier (18) pour translater un support (140) de levier axialement le long d'un axe longitudinal (X) du corps (26) de l'instrument chirurgical ;
interconnexion du premier levier (18) et du deuxième levier (46) avec un ressort (66) qui permet un mouvement du deuxième levier (46) en même temps que le premier levier (18) lorsque le premier levier (18) est déplacé jusqu'à une position qui résulte en la fermeture des mâchoires (14) de l'instrument chirurgical mais empêche un mouvement du deuxième levier (46) lorsque le premier levier (18) est déplacé au-delà de la fermeture des mâchoires (14) de telle sorte qu'un mouvement du premier levier (18) au-delà de la position résulte en la fermeture des mâchoires (14),
dans lequel le premier levier (18) et le deuxième levier (46) sont interconnectés par une liaison (60, 62) qui est connectée pivotante aux deux parmi le premier levier (18) et le deuxième levier (46), et
dans lequel le ressort (66) est configuré pour pousser le premier levier (18) et le deuxième levier (46) l'un vers l'autre.
